# EUROPEAN PATENT APPLICATION

(11) **EP 4 666 971 A1**
(43) Date of publication of application: **24.12.2025**
(21) Application number: 24909543.1
(22) Date of filing: 30.05.2024
(51) Int. Cl.: A61B 18/04, A61B 18/00

(54) **ANTI-BREAKDOWN RADIO FREQUENCY PLASMA SURGICAL ELECTRODE**

(30) Priority: 25.12.2023 CN 202323566763 U
(71) Applicant: Bangshi Medical Technology Co., Ltd., Taizhou, Jiangsu 225300 (CN)
(72) Inventor: HE, Chengdong, Taizhou, Jiangsu 225300 (CN); JIN, Liangcui, Taizhou, Jiangsu 225300 (CN); YUE, Xin, Taizhou, Jiangsu 225300 (CN)
(74) Representative: Bayramoglu et al.
(86) International application number: PCT/CN2024/096231
(87) International publication number: WO 2025/138577

(57) **Abstract**

The present utility model relates to the technical field of medical appliances, and in particular to an anti-breakdown radiofrequency (RF) plasma surgical electrode. The anti-breakdown RF plasma surgical electrode includes a cutter head and a cutter shank, where the cutter head includes a ceramic head, an electrode plate, and an electrode wire; the ceramic head is integrally formed, and includes a suction tube connection hole, a suction through hole, an electrode wire clamping hole, and a mounting groove; the suction tube connection hole is communicated with the suction through hole; an opening of the suction through hole and an opening of the electrode wire clamping hole penetrate through a front end surface of the ceramic head; the front end surface of the ceramic head is provided with the electrode plate; the electrode wire passes through the electrode wire clamping hole and is conductively connected to the electrode plate; and the cutter shank is insulated from the electrode plate. In the present utility model, the electrode wire of the electrode is completely arranged inside the integrally formed ceramic head, and a negative electrode tube is inserted into the ceramic head and completely isolated from the electrode wire and the electrode plate. Therefore, the present utility model avoids the possibility of electrode head breakdown caused to the existing surgical electrode by component displacement during the assembly of a plurality of components or during use, greatly improving the insulation performance of the electrode and the safety of surgery.

## Description

### TECHNICAL FIELD

The present utility model relates to the technical field of medical appliances, and in particular to an anti-breakdown radiofrequency (RF) plasma surgical electrode.

### BACKGROUND TECHNOLOGY

Recently, low-temperature plasma surgical systems have been widely used in otolaryngology, spine surgery, gynecology, proctology and other diseases abroad. The low-temperature plasma surgical system can precisely control the surgical temperature within 40-70°C, and features high safety, short surgical time, minimal trauma, and short postoperative recovery time. The basic principle of the plasma surgical system is as follows. Plasma energy flows between the working electrode and the loop electrode, and is conducted by physiological saline to form a highly concentrated plasma vapor sheath around the electrode. The plasma sheath includes a large number of charged particles. After being accelerated by the electric field, the charged particles generate sufficient energy and have strong oxidizing properties, breaking molecular bonds that make up the target tissue cells at low temperatures (40-70°C). Thus, the tissue rapidly decomposes into low-molecular-weight (LMW) molecules and atoms, resulting in real-time and efficient tissue cutting and ablation effects at lower temperatures.

As the closest prior art, the radiofrequency (RF) plasma surgical electrode (CN204219039U) includes a loop electrode and an active electrode that are located at an electrode head and isolated by a split insulator. The insulator inside the electrode head is likely to displace and cause an insulation error during the assembly (or use) of each part of the product, thereby posing a risk of breakdown caused to the loop electrode during use. Therefore, the safety of the electrode needs to be improved. In addition, the insulation performance of the electrode is poor, and the service life of the electrode is short.

### CONTENT OF THE INVENTION

The present utility model provides an anti-breakdown radiofrequency (RF) plasma surgical electrode, which solves the problem of an existing RF plasma electrode that has a risk of breakdown during use.

An anti-breakdown RF plasma surgical electrode includes a cutter head and a cutter shank, where the cutter head includes a ceramic head, as well as an electrode plate and an electrode wire arranged on the ceramic head;
the ceramic head is integrally formed, and includes a suction tube connection hole, a suction through hole, an electrode wire clamping hole, and a mounting groove;
the suction tube connection hole is communicated with the suction through hole; an opening of the suction through hole and an opening of the electrode wire clamping hole penetrate through a front end surface of the ceramic head; the front end surface of the ceramic head is provided with the electrode plate; the electrode wire is laid in the mounting groove; and a protruding end of the electrode wire passes through the electrode wire clamping hole and is conductively connected to the electrode plate; and
the cutter shank includes a negative electrode tube and a suction tube; the negative electrode tube is inserted into the ceramic head and insulated from the electrode plate.

Furthermore, the electrode plate is provided with a plurality of discharge blind holes and a plurality of fixing holes; the fixing hole corresponds to the electrode wire clamping hole; and a shape of the discharge blind hole includes square, triangle, circle, or strip.

Furthermore, the suction tube is inserted into the suction tube connection hole, and an outer diameter of the suction tube is adapted to an inner diameter of the suction tube connection hole.

Furthermore, the electrode wire is sleeved in an insulation sleeve.

Furthermore, the negative electrode tube is wrapped in an outer insulation layer.

Furthermore, the negative electrode tube is connected to a negative electrode wire; the electrode wire is connected to a positive electrode wire; and each of the negative electrode wire and the positive electrode wire is connected to an RF main body through a connector.

The present utility model has the following beneficial effects:
(1) In the present utility model, the electrode wire of the electrode is completely arranged inside the integrally formed ceramic head, and the negative electrode tube is inserted into the ceramic head and completely isolated from the electrode wire and the electrode plate. Therefore, the present utility model avoids the possibility of electrode head breakdown caused to the existing surgical electrode by component displacement during the assembly of a plurality of components or during use, greatly improving the insulation performance of the electrode and the safety of surgery.
(2) In the present utility model, the positive electrode is provided on an insulating shell made of ceramic, which has good insulation performance and prolongs the service life of the electrode.

### DESCRIPTION OF THE DRAWINGS

FIG. 1 is an overall appearance diagram of an electrode according to the present utility model;
FIG. 2 is an exploded view of the electrode according to the present utility model;
FIG. 3 is an axial sectional view of the electrode according to the present utility model;
FIG. 4 is a front side view of a ceramic head according to the present utility model; and
FIG. 5 is a rear side view of the ceramic head according to the present utility model.

Reference Numerals: 1. cutter head; 10. ceramic head; 101. suction tube connection hole; 102. suction through hole; 103. electrode wire clamping hole; 104. mounting groove; 11. electrode plate; 12. electrode wire; 2. cutter shank; 20. negative electrode tube; 21. suction tube; and 22. outer insulation layer.

### SPECIFIC IMPLEMENTATIONS

The principles and features of the present utility model are described below with reference to the drawings. The listed examples are only used to explain the present utility model, rather than to limit the scope of the present utility model.

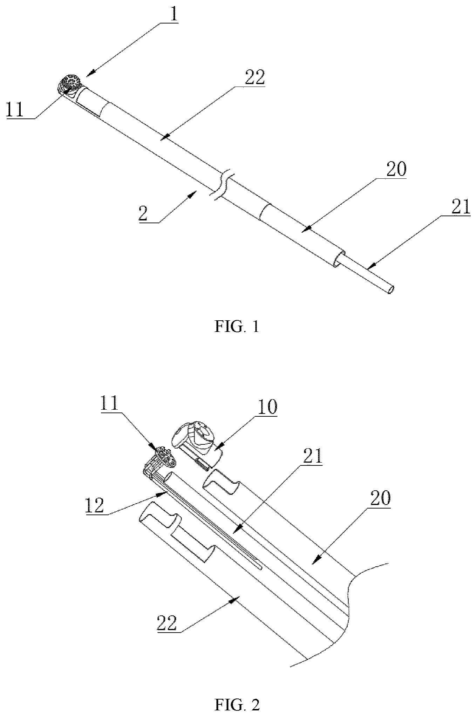

As shown in FIGS. 1 to 3, the present utility model provides an anti-breakdown radiofrequency (RF) plasma surgical electrode, including cutter head 1 and cutter shank 2. The cutter head 1 includes ceramic head 10, as well as electrode plate 11 and electrode wire 12 arranged on the ceramic head 10. The cutter shank 2 includes negative electrode tube 20 and suction tube 21.

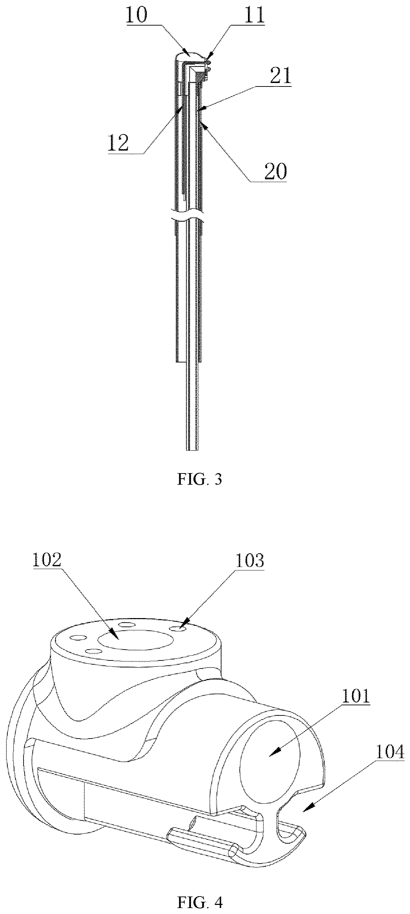

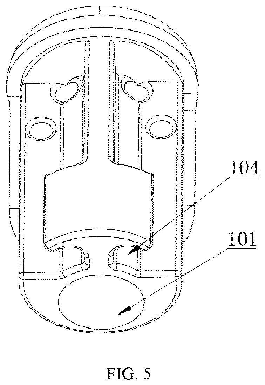

As shown in FIGS. 4 and 5, the ceramic head 10 includes a cylindrical body and a front end surface. The front end surface is perpendicular to the cylindrical body. The front end surface is also cylindrical. The cylindrical body is provided with axial suction tube connection hole 101 for mounting and fixing the suction tube 21. The cylindrical body is provided with arc-shaped mounting groove 104. The electrode wire 12 is laid along the mounting groove 104. A center of the front end surface of the ceramic head 10 is provided with suction through hole 102. The suction through hole 102 is connected to the suction tube connection hole 101. The suction tube 21 is inserted into and fixed inside the suction tube connection hole 101 to suck out a cut tissue and a waste liquid during a surgical process. A plurality of electrode wire clamping holes 103 are arranged around the suction through hole 102. The electrode wire clamping holes 103 are communicated with the mounting groove 104 to pass a protruding end of the electrode wire 12 out of the electrode wire clamping hole 103 and conductively connect the protruding end of the electrode wire to the electrode plate 11. One end of the electrode wire 12 forms the protruding end with a large diameter, which is clamped in a fixing hole of the electrode plate 11. The protruding ends of a plurality of electrode wires 12 provide the energy required during the surgical process and form a plasma cutting area through discharge.

Preferably, the electrode wire clamping hole 103 and the mounting groove 104 are at a 90° angle, and the electrode wire 12 is subjected to tension after being passed out, thereby preventing the electrode wire from falling off and ensuring safety during use. The arc-shaped mounting groove 104 can also pass the electrode wire 12 bent, playing a role in restraining and supporting the electrode wire 12, thereby preventing the electrode wire from falling off and touching the negative electrode tube 20, ensuring the safe insulation performance between the positive and negative electrodes.

Preferably, an outer diameter of the suction tube 21 is slightly smaller than an inner diameter of the suction tube connection hole 101, making it easier to insert the suction tube 21 into the suction tube connection hole 101 without leaking or loosening. The suction tube 21 is made of polyetheretherketone (PEEK), which is resistant to high temperatures and has a certain degree of elasticity.

Preferably, the electrode wire 12 is sleeved in an insulation sleeve to enhance insulation. The insulation sleeve is made of polyimide (PI), which has high insulation performance and can withstand high temperatures above 400°C.

The electrode plate 11 is provided with a plurality of discharge blind holes and a plurality of fixing holes. The fixing hole corresponds to the electrode wire clamping hole 103. The shape of the discharge blind hole includes square, triangle, circle, or strip, which provides a comprehensive and energy-rich discharge area on a surface of the electrode plate 11.

The ceramic head 10 is formed by integral casting, which simplifies the components required for electrode assembly and reduces the risk of electrode breakdown caused by assembly errors.

The negative electrode tube 20 is made of a metal material and has conductivity. The negative electrode tube is inserted outside the cylindrical body of the ceramic head 10 and has a large distance from the electrode plate 11, providing insulation and isolation. A side of the negative electrode tube 20 close to the electrode plate 11 is partially exposed to form a negative electrode of a loop.

The negative electrode tube 20 is wrapped in outer insulation layer 22, and the outer insulation layer 22 is also made of PI.

The negative electrode tube 20 is connected to a negative electrode wire. The plurality of electrode wires 12 are pressed together and connected to a positive electrode wire through welding. Each of the negative electrode wire and the positive electrode wire is connected to an RF main body through a connector.

In the present utility model, during electrode assembly, the electrode wire 12 passes through the electrode plate 11, and the insulation sleeve is fitted outside the electrode wire 12. The electrode wire 12 passing through the electrode plate 11 passes through the electrode wire clamping hole 103 of the ceramic head 10, bent 90°, and fixed in the mounting groove 104. The suction tube 21 is inserted into the suction tube connection hole 101, and the negative electrode tube 20 with the outer insulation layer 22 is fitted onto the ceramic head 10.

The above described is merely a preferred embodiment of the present utility model, but this embodiment does not represent all possible forms of the present utility model. Therefore, the protection scope of the present utility model is not limited to such special statement and embodiment. Various other modifications and improvements made based on the technical insights of the present utility model without departing from the essence of the present utility model still fall within the protection scope of the present utility model.

## Claims

1. An anti-breakdown radiofrequency (RF) plasma surgical electrode, comprising a cutter head (1) and a cutter shank (2), **characterized in that** the cutter head (1) comprises a ceramic head (10), as well as an electrode plate (11) and an electrode wire (12) arranged on the ceramic head (10);
the ceramic head (10) is integrally formed, and comprises a suction tube connection hole (101), a suction through hole (102), an electrode wire clamping hole (103), and a mounting groove (104);
the suction tube connection hole (101) is communicated with the suction through hole (102); an opening of the suction through hole (102) and an opening of the electrode wire clamping hole (103) penetrate through a front end surface of the ceramic head (10); the front end surface of the ceramic head (10) is provided with the electrode plate (11); the electrode wire (12) is laid in the mounting groove (104); and a protruding end of the electrode wire (12) passes through the electrode wire clamping hole (103) and is conductively connected to the electrode plate (11); and
the cutter shank (2) comprises a negative electrode tube (20) and a suction tube (21), wherein the negative electrode tube (20) is inserted into the ceramic head (10) and insulated from the electrode plate (11).

2. The anti-breakdown RF plasma surgical electrode according to claim 1, **characterized in that** the electrode plate (11) is provided with a plurality of discharge blind holes and a plurality of fixing holes; the fixing hole corresponds to the electrode wire clamping hole (103); and a shape of the discharge blind hole comprises square, triangle, circle, or strip.

3. The anti-breakdown RF plasma surgical electrode according to claim 1, **characterized in that** the suction tube (21) is inserted into the suction tube connection hole (101), and an outer diameter of the suction tube (21) is adapted to an inner diameter of the suction tube connection hole (101).

4. The anti-breakdown RF plasma surgical electrode according to claim 1, **characterized in that** the electrode wire (12) is sleeved in an insulation sleeve.

5. The anti-breakdown RF plasma surgical electrode according to claim 1, **characterized in that** the negative electrode tube (20) is wrapped in an outer insulation layer (22).

6. The anti-breakdown RF plasma surgical electrode according to any one of claims 1 to 5, **characterized in that** the negative electrode tube (20) is connected to a negative electrode wire; the electrode wire (12) is connected to a positive electrode wire; and each of the negative electrode wire and the positive electrode wire is connected to an RF main body through a connector.
